# EUROPEAN PATENT SPECIFICATION

(11) **EP 0 417 290 B1**
(45) Date of publication and mention of the grant of the patent: **11.12.1996**
(21) Application number: 89911892.1
(22) Date of filing: 26.10.1989
(51) Int. Cl.: A61N 1/30

(54) **INTERFACE FOR ELECTRIC ENDERMISM**
SCHNITTSTELLE FÜR ELEKTRISCHE ENDERMOSE
INTERFACE POUR ENDERMOSE ELECTRIQUE

(30) Priority: 26.10.1988 JP 268248/88; 26.05.1989 JP 131635/89; 12.06.1989 JP 146767/89
(43) Date of publication of application: 20.03.1991
(73) Proprietor: HISAMITSU PHARMACEUTICAL CO. INC., Tosu-shi Saga 841 (JP)
(72) Inventor: OKABE, Keiichirou, Tokyo 157 (JP); TOCHIKUBO, Osamu, Yokohama-shi Kanagawa 240 (JP)
(74) Representative: Cohausz & Florack Patentanwälte
(86) International application number: JP8901101
(87) International publication number: WO9004434

(56) References cited:
- JP-A-63 102 768
- JP-B- 0 059 224
- JP-U-52 168 494
- JP-Y- 5 530 482
- US-A- 4 164 226
- US-A- 4 506 680
- US-A- 4 606 118
- US-A- 4 764 379
- PATENT ABSTRACTS OF JAPAN vol. 13, no. 494 (C-651)8 November 1989 & JP-A-1 197 429 ( OLYMPUS OPTICAL )
- PATENT ABSTRACTS OF JAPAN vol. 6, no. 63 (C-99)22 April 1982 & JP- A-57 004 915 ( FUJISAWA YAKUHIN KOGYO )

## Description

### TECHNICAL FIELD

The present invention relates to a skin contact interface (skin contacting member) useful for an electrical percutaneous drug administration (iontophoresis, etc.).

### BACKGROUND ART

Increasing developments have been made in the use of an iontophoresis as an active percutaneous drug administration system.

However, the relationship between living body channels (sudoriferous glands, etc.) estimated to be utilizable for a percutaneous permeation of high molecular drugs such as peptides, i.e., epidermal fine pores, and for a percutaneous drug administration, has not been sufficiently clarified under the present technical state of the art.

The structure of an interface for iontophoresis comprises a reservoir for holding a drug solution and electrodes for current dispersion. The structure of the reservoir must allow a predetermined amount of the drug solution to reach the living body skin interface, with a lapse of time, but the reservoir itself is three-dimensional, and further, water is used as the medium, and thus a problem such as a dilution of the drug arises.

An interface is known from US-A-4164226 wherein an iontophretic burn-protection electrode structure is disclosed in which the porous intervenor material serving as reservoir for the drug solution (e.g., felt) is composed of metal-free natural fibers of wool or cotton or eqivalent man-made fibrous materials such as viscose, rayon or polyester.

Furthermore US-A-4606118 reveals an electrical stimulation lead, in which porous glas, ceramic, plastic or metal is used as reservoir for the drug solution.

### DISCLOSURE OF THE INVENTION

In view of the state of the art as mentioned above, the object of the present invention is to provide an interface suitable for iontophoresis and having a structure capable of performing an accurate and safe drug administration.

Other objects and advantages of the present invention will be apparent from the descriptions hereinbelow.

In accordance with the present invention, there is provided an interface for iontophoresis comprising:
- a porous material having a dried drug arranged on the surface thereof to be contacted with the skin,
- a reservoir for containing a conveying liquid and
- a hollow needle providing a contact between said reservoir and said porous material for permitting said conveying liquid to permeate through said porous material.

### BRIEF DESCRIPTION OF THE DRAWINGS

The present invention will now be further explained in detail with reference to the drawings.
Figure 1 to Fig. 5 are drawings illustrating examples of technical aspects of interfaces for iontophoresis;
Fig. 6 and Fig. 7 are drawings illustrating Examples of the present invention.

### BEST MODE OF CARRYING OUT THE INVENTION

According to the present invention, an electroconductive channel is primarily formed by filling a liquid into living body channels such as sudoriferous glands through the electrical permeation effect of the interface. Generally, a human being has 2,000,000 to 5,000,000 sudoriferous glands, each being a single tubular gland or gland body bent in a corium or subcutaneous binding tissue to form a glomerular shape, and having a spiral discharge tube opening on the surface of the epidermis (sudoriferous pore).

Also, fine smooth muscle fibers surround the gland body outer surface, and these are surrounded by a dense capillary net formed by branchings from skin arteries. Therefore, it is apparent that sudoriferous glands are utilizable as an electrical electroconductive living body channel, and that they provide the shortest route for conveying a drug solution to the blood.

Nevertheless, although the sudoriferous glands are pores opening outwardly, as described above, generally a gas with a high electrical impedance, such as air and other gases, exists in the vicinity of the discharge tubes of the sudoriferous pores.

Particularly, when electrodes used to apply a current to a living body, for various purposes, are brought into contact with the living body, since a gas is sandwiched between the electrodes and the living body, sudoriferous glands at the point where intervening gas exists are given a high electrical impedance, and thus cannot substantially contribute to the iontophoresis of drugs, as a non-electroconductive channel.

Electrical permeation refers to the phenomenon whereby, when a liquid is divided into two compartments with a substance which forms a capillary or porous structure, and a direct current is applied thereto by placing electrodes in both liquids, the liquid moves. The direction of movement of the liquid is determined in accordance with the sign of the ε-potential between the liquid and the substance.

Accordingly, the subject matter of the present invention is to constitute an interface (interface forming means) comprising a non-electroconductive substance having a capillary or porous structure and carrying a drug and a conveying liquid, bring the interface forming means into contact with living body skin tissue surface to connect the fine pore portion of the interface forming means with sudoriferous glands, form an electroconductive channel by filling the liquid in the sudoriferous glands by applying a direct current or pulsed voltage thereto to obtain the electrical permeation effect, and subsequently, carrying out the electrical percutaneous drug administration such as a conventional ion introduction method (iontophoresis).

The porosity of the above-mentioned interface forming means may be suitably selected depending on the purpose, but in general is set so that the sudoriferous glands of the living body and the pore portions of the interface forming means are in good communication with each other, and is preferably 10% to 90%.

The interface forming means is used at the anode side or the cathode side, depending on whether the zeta potential of the fine pores thereof during charging is plus or minus.

For example, if the interface forming means is positively charged, the liquid will be negatively charged, and thus the interface forming means is arranged on the cathode side. On the other hand, if the interface forming means is negatively charged, the liquid will be positively charged, and thus the interface forming means is arranged and used on the anode side.

According to a constructional aspect of such an interface, an unevenness is further formed on the skin contacting surface. This unevenness may be suitably chosen with respect to the shape, distribution density, and height of the unevenness at the skin contacting site, and is not particularly limited. Also, if desired, fine particles with sharp angular shapes, such as fine glass particles, may be arranged on the unevenness portion surface so that fine cracks can be formed in the corneum.

According to an embodiment of the present invention, a solid or dry drug is coated or attached to on one surface of a porous material, and by applying a current and supplying a conveying liquid from the other surface during use, the conveying liquid and the drug are mixed together only during use to form a locally highly concentrated drug solution. Accordingly, the administration of a percutaneous drug solution is promoted while maintaining a highly concentrated solution without a diffusion dilution of the drug solution by the electrical force.

Also, the present invention is suitable for the storage of a drug for long term, because the drug before use is a solid, i.e., is in the dry state.

Examples of the porous material usable in the present invention include porous materials made of ceramics such as bisque, alumina, and zirconia, or synthetic resin materials. The average pore size is preferably several µm to several hundred µm, and the porosity is preferably 10 to 90%. Both the pore size and porosity may be selected in accordance with the number of sudoriferous glands to which the dose is to be applied and the drug dose to be used, and is not particularly limited.

Also, ceramics materials and synthetic resin materials which are worked by laser to form capillary structures can be used. The thickness of these materials is not particularly limited, but is preferably 0.1 mm to 10 mm.

In some cases, a flexible film or sheet materials may be employed, provided that the capillary is non-deformable.

Examples of the conveying liquid shown in the present invention include water and electrolyte solutions such as sodium chloride, but these do not limit the invention.

Examples of the structure and actuation of exemplary interfaces for iontophoresis are now described with reference to the drawings.

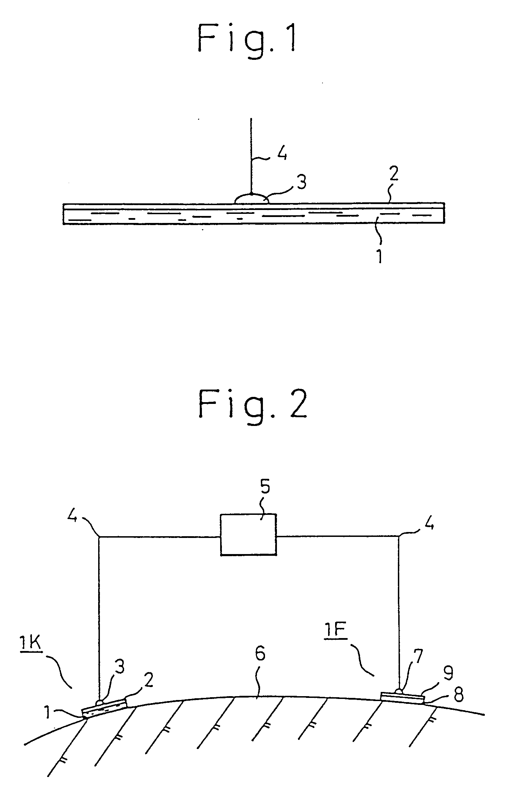

Figure 1 shows an example of an interface for iontophoresis, wherein (1) is an interface forming means comprising a ceramics material or a non-electroconductive material such as a synthetic resin material, and having a capillary or porous (continuous pore) structure. The average pore size in the capillary or porous structure is generally 0.01 to 500 µm, preferably 0.01 to 10 µm, and the porosity is preferably 10 to 90%, more preferably 30 to 90%. Both the pore size and porosity may be selected in accordance with the number of sudoriferous glands to which it is to be applied and the drug dose to be used, and is not particularly limited.

Here, ceramics materials refer to all porous materials produced in the ceramic field, such as porous alumina and bisque, etc.

On the other hand, the synthetic resins materials may be those having an electrical permeation effect due to a porous or capillary structure, for example, propylene, polyethylene, vinyl chloride, and silicone, and are not particularly limited.

Also, ceramics materials and synthetic resin materials which are worked by laser to form capillary structures can be used. The thickness of these materials is not particularly limited, but is preferably 0.1 mm to 10 mm.

In Fig. 1, (2) is an electrode comprising an electroconductive material such as a metal or carbon. In Fig. 1, the electrode (2) is shown as a single layer structure, but a structure having a reservoir containing a drug solution for iontophorese interposed therebetween, and further, having the above electroconductive member laminated thereon, may be used. Further, a conventional gel-like electrode for a living body may be used as the electrode (2).

The electrode (2) is provided with a connector (3) for connection with an electrical lead wire (4) from a power supply unit (3) including an external battery power source.

Also, by binding the above-mentioned miniaturized power supply unit to the electrode (2), and further imparting a tackiness to the interface forming means or providing a tacky layer therearound, the whole may be constituted so as to be plasterable onto the skin.

Rigid materials are preferably used for the interface forming means, but in some cases (namely, if the capillary is non-deformable), a flexible film or sheet material may be used.

An embodiment of this exemplary interface for iontophores comprising the above constitution is now described in detail.

Figure 2 shows an anode portion (1K) having the structure shown in Fig. 1, a cathode portion (1F) having a laminated structure of an electroconductive tacky gel portion (8) and an electrode (9) and a power source such as battery constituting a power supply unit (5) for outputting a direct current or pulsed voltage, and a lead wire (4) for connecting the cathode portion (1K) and the cathode portion (1F) to the power supply unit (5).

In Fig. 2, the anode portion (1K) and the cathode portion (1F) are shown in contact with a living body skin surface (6).

In Fig. 2, after the interface forming means (1) is brought into contact with a living body skin surface, a direct current or pulsed voltage is output from the power supply unit. The interface forming means (1) receives the voltage and thereby moves the liquid as a positively charged drug solution toward the sudoriferous glands, the liquid penetrates the sudoriferous glands, and the sudoriferous glands become electroconductive channels connecting the inside and outside of the living body, and as a result, a subsequent electrical percutaneous drug administration process such as iontophoresis can be effectively accomplished.

As described in detail above, such an interface for iontophoresis enables an electrical percutaneous drug administration through sudoriferous glands by making electroconductive channels of the sudoriferous glands by filling a liquid therein by an electrical permeation effect, and further, making a good electroconductive channel between the external power source and living body skin tissue, and therefore, a superfluous polarization does not occur, and there is no danger of, for example, burning.

Next, an experimental example of such an interface for iontophoresis is described in detail with reference to the drawing.

The interface forming means was made from a bisque material with a 60% porosity and having a size and thickness about the same as a 10-yen coin (i.e., in the shape of a disc having a diameter of about 20 to 25 mm and a thickness of about 1 mm). The interface forming means was impregnated with an aqueous 10% redocaine hydrochloride solution, and a carbon material sheet was employed as the electrode.

The pulse depolarization system iontophoresis disclosed by Japanese Unexamined Patent Publication (Kokai) No. 58-159076, (outputting a depolarized pulse wave with a pulse wave height value of 12 V, a frequency of 40 kHz, and a duty of 3%) was used as the power supply unit, and was connected through tee output side (+) of the power supply unit and electroconductive wire to the connector of the above electrode. A gel-like living body electrode for ECG was connected to the other output side.

The interface forming means and the ECG electrode were brought into intimate contact with a human right upper arm portion, at a spacing therebetween of 5 cm, and the (+) contact portion was pricked with a needle at predetermined intervals, to confirm the extent of the presence of subcutaneous anesthesia.

Further, a defatted cotton impregnated with an aqueous 10% ridocaine hydrochloride as the interface forming means was brought into contact with a human left upper arm portion a spacing of 5 cm, and using the same power supply unit as described above, and the same stimulation, the extent of the presence of subcutaneous anesthesia was measured.

As a result, when a bisque material was used as the interface forming means, it was confirmed that a strong subcutaneous anesthesia was exhibited within 6 minutes, but when a defatted cotton was used as the interface forming means, only a weak anesthesia was recognized even after an elapse of 20 minutes.

If the above interface forming means is used on the (-) side, it becomes possible to aspirate a body fluid, whereby a living body sensor of the non-invasion type can be constituted, and the present invention also includes this concept.

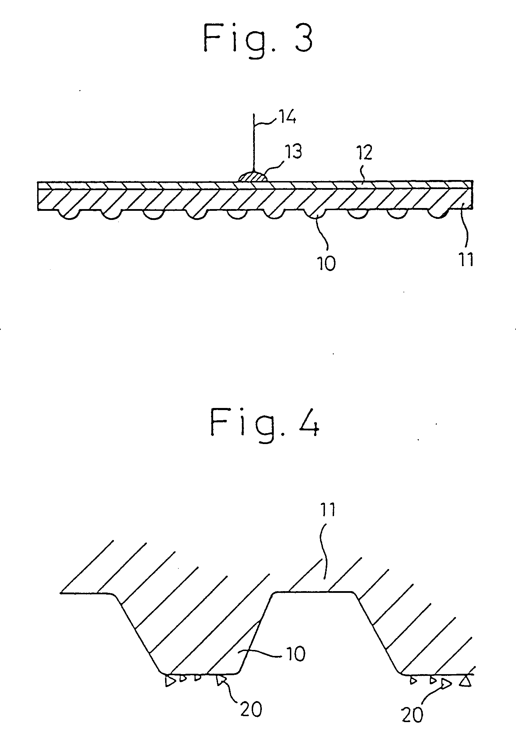

Figure 3 is a drawing illustrating an example of a constructional aspect of an interface for iontophoresis. In Fig. 3, (11) is an interface forming means comprising a ceramics material or a non-electroconductive material such as a synthetic resin material, and having a capillary or porous (continuous pore) structure. A plurality of unevennesses (10) are formed on the skin contacting interface, integrally with the interface forming means (11). The average pore size in the capillary or porous structure is generally 0.01 to 500 µm, preferably 0.1 to 1 µm, and the porosity is generally 10 to 90%, preferably 30 to 90%. Both the pore size and porosity may be selected in accordance with the number of sudoriferous glands to which it is to be applied and the drug dose to be used, and is not particularly limited.

Here, ceramics materials refer to all porous materials produced in the ceramic field, such as porous alumina and bisque, etc.

Further, the synthetic resins materials may be those having an electrical permeation effect due to a porous or capillary structure, for example, propylene, polyethylene, vinyl chloride, and silicone, and are not particularly limited.

Also, ceramics materials and synthetic resin materials which are worked by laser to form capillary structures can be used. The thickness of these materials is not particularly limited, but is preferably 0.1 mm to 10 mm.

(12) is an electrode comprising an electroconductive material such as a metal or carbon. In Fig. 3, the electrode (12) is shown as a single layer structure, but a structure having a reservoir containing a drug solution for iontophorese interposed therebetween, and having the above electroconductive member laminated thereon, may be used. Further, a conventional gel-like electrode for a living body may be used as the electrode (12).

The electrode (12) is provided with a connector (13) for connection with an electrical lead wire (14) from a power supply unit (13) including an external battery power source.

Also, by binding the above-mentioned miniaturized power supply unit to the electrode (12), and further, imparting a tackiness to the interface forming means or providing a tacky layer therearound, the whole may be constituted so as to be plasterable onto the skin.

Rigid materials are preferably used for these interface forming means, but in some cases (namely, if the capillary is non-deformable), a flexible film or sheet material may be used.

In Fig. 4, another example of the constructional aspect is shown.

Figure 1 shows an example comprising rigid fine particles having a sharp angularity composed of, for example, glass attached to or coated on the unevennesses (10) formed on the interface forming means (11). Since this otherwise has the same shape or structure as shown in Fig. 3, the portion having the rigid fine particles arranged is enlarged in the drawing, and other portions are omitted. The material of the rigid fine particles is not particularly limited.

An embodiment of an interface for iontophoresis comprising the above constitution is now described in detail.

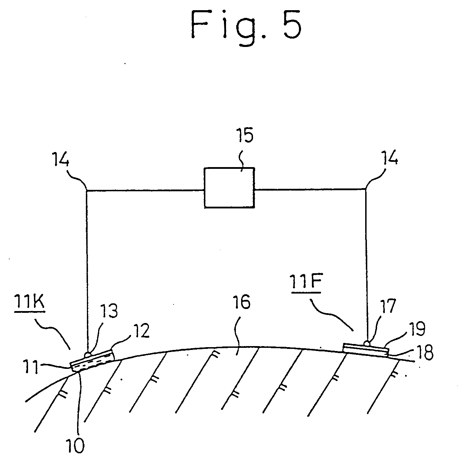

Figure 5 shows an anode portion (11K) having the structure shown in Fig. 3, a cathode portion (11F) having a laminated structure of an electroconductive tacky gel portion (18), an electrode (19), and a power source such as battery constituting a power supply unit (15) for outputting a direct current or pulsed voltage, and a lead wire (14) for connecting the cathode portion (11K) and the cathode portion (11F) with the power supply unit (15).

In Fig. 5, the anode portion (11K) and the cathode portion (11F) are shown in contact with a living body skin surface (16). The interface forming means (11) is brought into intimate contact with the skin surface (16) through the unevennesses provided on the surface thereof.

In Fig. 5, after the interface forming means (11) is brought into contact with a living body skin surface, a direct current or pulsed voltage is output from the power supply unit. The interface forming means (11) receives the voltage and thereby moves the liquid as a positively charged drug solution toward the sudoriferous glands, the liquid penetrates the sudoriferous glands, and the sudoriferous glands become electroconductive channels connecting the inside and outside of the living body, and as a result, a subsequent electrical percutaneous drug administration process such as iontophoresis can be effectively accomplished.

On the other hand, in the example shown in Fig. 5, when the interface forming means (11) is brought into contact with the skin surface (16), a tension on the skin surface is caused by the unevennesses (10), and this tension causes the rigid particles arranged on the unevennesses (10) to move and thereby cause microscopic damage to the skin surface. This causes cracks to form in the corneum, i.e., the skin surface barrier, and thus the introduction of a drug solution, particularly the introduction of a drug solution with a large molecular weight, is facilitated.

As described in detail above, such an embodiment of an interface for iontophoresis enables an electrical percutaneous drug administration through sudoriferous glands by making electroconductive channels of the sudoriferous glands by filling a liquid therein by the electrical permeation effect, and further, makes a good electroconductive channel between the external power source and living body skin tissue, and therefore, has an effect such that no superfluorous polarization occurs, and that there is no danger of, for example, burning.

Next, an experimental example of such an embodiment is described in detail with reference to the drawing.

The interface forming means was made from a bisque material with a 60% porosity and in the shape of a disc having a diameter of about 2 cm and a thickness of about 1 mm. The bisque material had about 10 unevennesses with a height of about 2 mm equally distributed thereon. The interface forming means was impregnated with an aqueous 10% ridocaine hydrochloride solution, and a carbon material sheet was employed as the electrode.

The pulse depolarization system iontophoresis disclosed by Japanese Unexamined Patent Publication (Kokai) No. 58-159076 (outputting a depolarized pulse wave with a pulse wave height value of 12 V, a frequency of 40 kHz, and a duty of 3%) was used as the power supply unit, and was connected through the output side (+) of the power supply unit and electroconductive wire to the connector of the above electrode. A gel-like living body electrode for ECG was connected to the other output side.

The interface forming means and the ECG electrode were brought into intimate contact with a human right upper arm portion of a spacing therebetween of 5 cm, and the (+) contact portion was pricked with a needle at predetermined intervals, to confirm the extent of the presence of subcutaneous anesthesia.

Further, a defatted cotton impregnated with an aqueous 10% ridocaine hydrochloride as the interface forming means was brought into contact with a human left upper arm portion at a spacing of 5 cm, and using the same power supply unit as described above, and the same stimulation, the extent of the presence of subcutaneous anesthesia was measured.

As a result, when a bisque material was used as the interface forming means, it was confirmed that a strong subcutaneous anesthesia was exhibited within 6 minutes, but when a defatted cotton was used as the interface forming means, only a weak anesthesia was recognized even after an elapse of 20 minutes.

If the above interface forming means is used on the (-) side, it becomes possible to aspirate a body fluid, whereby a living body sensor of the non-invasion type can be constituted, and the present invention also includes this concept.

Next, example structures and functions of the present invention will now be explained in detail with reference to the drawings.

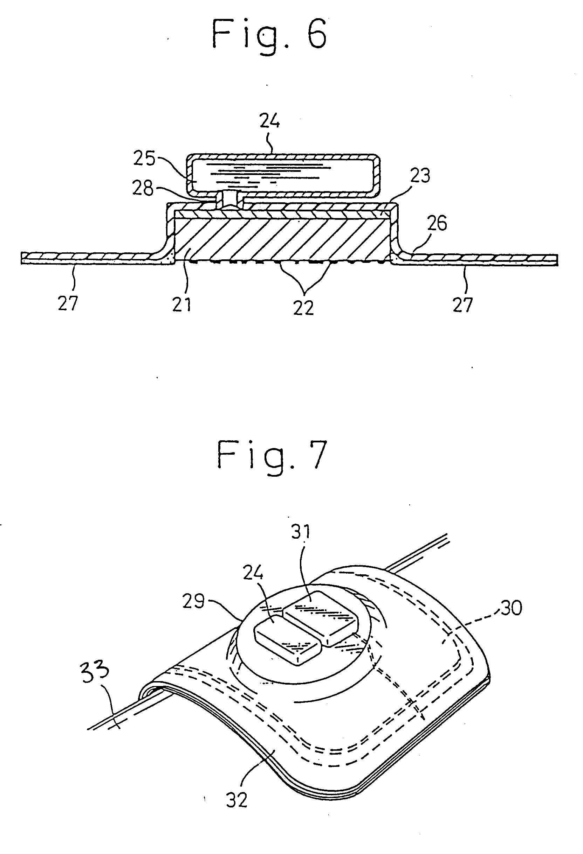

In Fig. 6, (21) is a ceramics porous member (the material thereof is not particularly limited) having a porosity set within the range mentioned above, and a dried drug is spray coated on one surface of the porous member (21) to form a drug particle attached surface (22).

Further, (23) is an electroconductive member comprising an electroconductive rubber, an electroconductive polymer, a carbon film, an aluminum foil, and a metal foil. The electroconductive member (23) may also comprise a porous material and may have a porosity selected as desired, but the porosity must exist to an extent such that the conveying liquid is permeated therethrough when a conveying liquid is injected.

The laminated structure of the porous member (21), drug particle attached surface (22), and electroconductive member (23) is covered with and thereby supported and fixed by, a flexible supporting member (26).

A plastering layer (27) is formed on the supporting member (26), for fixing the above-mentioned laminated structure to a living body skin surface.

Before use, the interface in the state as described above is placed on a paper coated with a silicone, and stored. During use, the drug particle attached surface (22) and living body surface are brought into contact, the reservoir (24) containing the conveying liquid (25) sealed therein is communicated by the penetration of a hollow needle (28) provided at the reservoir (24) from above the supporting member (26), and the conveying liquid (25) is supplied to the porous member (21) through the hollow needle (28) and the electroconductive member (23).

Next, a current is applied to the electroconductive member (23), whereby the conveying liquid (25) is permeated through the electroconductive member (23) and the porous member (21) until reaching the drug particle attached surface (22). Thereafter, the conveying liquid (25) is mixed with the drug particle attached surface (22) to become a liquid and a liquefied drug layer is formed on the living body skin surface, and further, the drug solution is permeated into living body through an electrical force.

Since the drug particle attached surface (22), which has become liquid, is permitted to move only toward the living body, a high drug concentration can be maintained without diffusion.

Figure 7 is a schematic illustration of the practical use of the plaster on a living body skin (33).

The laminate (29) consisting of the porous member, the electroconductive member and the drug layer is covered with the supporting member (34), and these elements are plastered to the skin (33) by the plastering agent layer provided on the supporting member (34).

Further, (24) is a reservoir, shown in the state in which it is bound to the porous member by a needle penetration, and (31) is a power supply unit comprising a battery and a chip type electronic circuit.

Also, in addition to the plastering agent layer, a counter-electrode layer (32) comprising an electroconductive member at the outermost periphery thereof is formed on the supporting member (30).

The above-mentioned plastering agent layer endowed with an electroconductivity is laminated on the surface of the counter-electrode layer (32), and the counter-electrode layer (32) is connected to the counter-electrode output terminal of the power supply unit (31).

During the application of a current between the electroconductive member (23) shown in Fig. 6 and the counter-electrode layer (32) shown in Fig. 7, the current passes through the porous member (21), the drug particle attached surface (22), the living body skin (33), and the electroconductive plastering layer, and thus the drug in the drug layer is liquefied by mixing the conveying liquid, and carried by the conveying liquid to penetrate the living body skin.

The electric output to be used in the present invention is a depolarized pulse which can perform a stable drug administration without irritation or pain, even if the current passage is not satisfactory, as shown in Japanese Unexamined Patent Publication (Kokai) No. 60-156475.

Not only the above-mentioned output but also an alternate current or direct current can be used, depending on the use mode, and this does not limit the invention.

Also, the example shown in Fig. 7 shows a structure wherein the power supply unit is integrally mounted thereto, but this is not critical and the power supply unit may be separated therefrom and connected thereto with a lead wire, or the shape may be changed depending on the use mode.

The molecular weight and other various amounts of the above-mentioned drug layer are not limited, but the interface of the present invention is particularly useful for peptide type drugs such as insulin, which maintains as high a concentration as possible and requires the presence of a sufficient amount of water to conduct an efficient iontophoresis even with a minute dosage amount. Examples of the drugs are shown below.

### Antitussive expectorants

sodium chromoglycate, ketotiphen fumarate

### Bronchodilators

hormoterol fumarate

### Analgesics

nalbufin hydrochloride, bentazocin lactate, cyclophenac sodium,

### Cardiacs

dopamine hydrochloride

### Psychoneurotic stabilizers

perphenazine, phenothiazine

### Antibiotics

cefotetan disodium, dibekacin sulfate, amikacin sulfate, netylmanocin sulfate, sisomycin sulfate

### Anti-malignant tumor agents

adriamycin, mitomycin C, bleomycin hydrochloride, lentinan, picivanil, bincrystine sulfate, cisplatin

### Circulatory function improvers

nikametate citrate, meclofenoxate hydrochloride, lislid maleate, calcium hopatenoate

### Gout therapeutics

allopurinol

### Other peptides

LHRH, enchephalin, endorphin, interferon, insulin, calcitonin, TRH, oxytocin, lypressin, vasopressin, glucagon, pituitary hormones (HGH, HMG, HCG, desmopressin acetate), and follicular luteinizing hormone.

These dried drug layers can be formed by various methods, such as spray coating or dipping followed by drying, and this is not particularly critical.

As described above in detail, the present invention can supplement an appropriate amount of water without a dilution of the drug solution, and since the drug before use is in the dry state, no denaturation or putrefaction occurs and a prolonged storage thereof is possible. Moreover, since the drug exists only at the surface in contact with the skin, and a diffusion in directions other than toward living body is impeded by the electrical force, the invention has the effect of performing a required drug administration without wastage of the drug solution.

### LIST OF REFERENCE NUMERALS

- 1, 11: Interface forming means,
- 2, 12: Electrode,
- 3, 13: Connector,
- 4, 14: Electrical lead wire,
- 5, 15: Power supply unit,
- 6, 16: Living body skin surface,
- 7, 17: Connector,
- 8, 18: Electroconductive tacky gel portion,
- 9, 19: Electrode,
- 1K, 11K: Anode portion,
- 1F, 11F: Cathode portion,
- 10: Unevenness,
- 20: Rigid fine particles,
- 21: Porous member (porous material),
- 22: Drug particle attached surface,
- 23: Electroconductive member,
- 24: Reservoir,
- 25: Conveying liquid,
- 26: Supporting member,
- 27: Plastering agent,
- 28: contracting means,
- 29: Laminate,
- 30: Supporting member,
- 31: Power supply unit,
- 32: Counter electrode layer,
- 33: Living body skin

## Claims

1. An interface for iontophoresis comprising:
- a porous material (21) having a dried drug (22) arranged on the surface thereof to be contacted with the skin (33),
- a reservoir (24) for containing a conveying liquid (25) and
- a hollow needle (28) for providing a contact between said reservoir (24) and said porous material (21) for permitting said conveying liquid (25) to permeate through said porous material (21).

2. An interface according to claim 1, wherein the porous material (21) is a ceramics or synthetic resin material.

3. An interface according to claim 1, wherein the average pore size of the porous material (21) is 0.01 to 500 µm.

4. An interface according to claim 1, wherein the porosity of the porous material (21) is 10 to 90%.

5. An interface according to claim 1, wherein the drug is antitussive expectorance, bronchodilators, analgesics, cardiacs, psychoneurotic stabilizers, antibiotics, antimalignant tumor agents, circulatory function improvers, gout therapeutics, or peptides.

## Patentansprüche

1. Schnittstelle zur Iontophorese mit
- einem porösen Material (21), auf dessen mit der Haut (33) in Kontakt zu bringenden Fläche sich ein getrocknetes Arzneimittel (22) befindet,
- einem Speicher (24) für eine Förderflüssigkeit (25) und
- einer Hohlnadel (28), um einen Kontakt zwischen dem Speicher (24) und dem porösen Material (21) herzustellen, damit die Förderflüssigkeit (25) durch das poröse Material (21) dringen kann.

2. Schnittstelle nach Anspruch 1, bei der das poröse Material (21) ein Keramikmaterial oder ein Kunstharzmaterial ist.

3. Schnittstelle nach Anspruch 1, bei der die durchschnittliche Porengröße des porösen Materiales (21) 0,01 bis 500 µm beträgt.

4. Schnittstelle nach Anspruch 1, bei der die Porosität des porösen Materiales (21) 10 bis 90 % beträgt.

5. Schnittstelle nach Anspruch 1, bei der das Arzneimittel die folgenden Arzneimittel umfaßt: Antitussive Expektorantien, Bronchodilatoren, Schmerzmittel, Herzmittel, Psychoneurosestabilisatoren, Antibiotika, Mittel gegen bösartige Tumoren, Mittel zur Verbesserung der Kreislauffunktion, Mittel gegen Gicht oder Peptide.

## Revendications

1. Interface pour l'iontophorèse, comprenant :
- une matière poreuse (21) à la surface de laquelle est disposé un médicament séché (22) pour être en contact avec la peau (33),
- un réservoir (24) pour contenir un liquide de transport (25) et une aiguille creuse (28) pour établir un contact entre ce réservoir (24) et cette matière poreuse (21) afin de permettre à ce liquide de transport (25) de traverser cette matière poreuse (21) par perméation.

2. Interface selon la revendication 1, dans laquelle la matière poreuse (21) est une céramique ou une résine synthétique.

3. Interface selon la revendication 1, dans laquelle la taille moyenne de pore de la matière poreuse (21) est de 0,01 à 500 µm.

4. Interface selon la revendication 1, dans laquelle la porosité de la matière poreuse (21) est de 10 à 90%.

5. Interface selon la revendication 1, dans laquelle le médicament est un expectorant antitussif, un bronchodilatateur, un analgésique, un médicament pour le coeur, un stabilisant psychonévrotique, un antibiotique, un anticancéreux, un agent améliorant la fonction circulatoire, un médicament pour la goutte, ou un peptide.
